# EUROPEAN PATENT APPLICATION

(11) **EP 3 978 036 A1**
(43) Date of publication of application: **06.04.2022**
(21) Application number: 20206958.9
(22) Date of filing: 11.11.2020
(51) Int. Cl.: A61L 9/00, A61L 9/03

(54) **ELECTRIC HEATER DEACTIVATING AIRBORNE PATHOGENS**

(30) Priority: 02.10.2020 TR 202015729
(71) Applicant: MCC Medikal Ozel Saglik Hizmetleri Endustriyel Makine Bilgisayar Elektronik AR-GE LTD., Antalya (TR)
(72) Inventor: CANPOLAT, MURAT, ANTALYA (TR)
(74) Representative: Sevinç, Cenk

(57) **Abstract**

The invention relates to the use of an electric heater also as an airborne pathogen deactivation system. In particular, the invention provides the use of an electric heater designed to deactivate viruses, bacteria, and similar pathogens in closed environments while heating a closed environment as a result of exposing pathogens to high temperature while passing through the air tubes.

## Description

### Technical Field of the Invention

The present invention relates in general to air purification and, in particular, to air purification systems that enable the deactivation of viruses, bacteria, and similar airborne pathogens. The present invention has the specific advantage of being an air purification system as well as an electric heater to heat indoor environments.

The invention more particularly relates to an electric heater designed to deactivate viruses, bacteria, and similar pathogens in a closed environment. The device can be used both as a heater and as an air pathogen deactivator, which neutralizes pathogens by heating the air passing through it.

### Background of the Invention

Electric heaters emit infrared radiation. Typically, infrared radiation is reflected by a reflector into the environment to be heated. The interaction of the infrared radiation with substances in the environment increases the temperature of the air. Another type of electric heater heats the environment using a fan. These heaters have a fan on the front surface and resistors (thin wires) that radiate heat behind the fan. The fan at the front of the heater draws the air into it from the back side of the heater. The air hitting the thin wire resistors heats up and leaves the heater's front side and heats the environment.

Airborne pathogens cause upper and lower respiratory tract diseases when inhaled (Pablo Jeremías et al., 2013). Studies have been conducted to examine the inactivation of bacteria and viruses in liquid and air at different temperatures (Aboud, Altemimi, Al-hiiphy, Yi-chen, & Cacciola, 2019; Bertrand et al., 2012). Several groups have studied the inactivation of pathogens at different air temperatures. Since the invention relates to airborne pathogens' inactivation by heating the air, references are given below accordingly.

Viruses, bacteria, allergens, pollen, fungi, mold, etc. are aerosols in the air. Pathogen inactivation depends on the heated air temperature. At one temperature, one pathogen may be inactivated, while another may only slightly be affected. For example, while Escherichia Coli is entirely inactive at 160 °C, Bacillus Subtilis is partially inactive, but 99.9% inactive at 360 °C (Jung, Lee, & Kim, 2009). Additionally, it has also been shown that Aspergillus versicolor and Cladosporium cladosporioides are inactivated by 99% within 0.2 seconds at 350 and 400 °C (Jung, Lee, Lee, Kim, & Lee, 2009). The MS2 virus response to dry air temperature was also examined, and 99.996% was inactivated in less than one second at 250 °C (Grinshpun et al., 2010).

Several studies have been conducted to inactivate the SARS-CoV-2 coronavirus with dry air heat. One of these studies tested the sterilization of used masks by exposing them to hot air. A study conducted for this purpose showed that SARS-CoV-2 in the N95 mask was inactivated by 99.9% at 70 °C (Yap, Liu, Shveda, & Preston, 2020). It was also shown that SARS-CoV was completely ineffective after exposure to 75 °C for 45 minutes in a liquid (Darnell, Subbarao, Feinstone, & Taylor, 2004). A study examining the inactivation of SARS-CoV-2 with temperature showed that the virus in serum became completely ineffective by keeping it at 92 °C for 15 minutes (Pastorino, Touret, Gilles, & Lamballerie, 2020). It has been shown that SARS-CoV and SARS-COV-2 inactivation rates with temperature were the same. For both viruses, a 5log-reduction was obtained at temperatures of 60 °C, 80 °C and 100 °C in 32.5, 3.7, and 0.5 minutes, respectively. Both viruses were deactivated at 120 °C with a 5log-decline in 5.4 seconds (Hessling, Hoenes, & Lingenfelder, 2020). In another study investigating airborne pathogens' inactivation at very high temperatures, E. Coli and MS2 virus were used. This study showed that both pathogens were inactivated at a rate of 4.7log in 0.41 seconds at 450 oC (Damit, Wu, & Yao, 2013).

The above studies have shown that airborne pathogens can be inactivated under high temperatures within seconds. However, an electrical heating system that inactivates airborne pathogens by increasing air temperature while passing through it has not been encountered in the literature.

As a result, in order to prevent the spread of SARS-CoV-2, influenza, and similar viruses and bacterial outbreaks, it was deemed necessary to re-design electric heaters used for indoor heating environments to neutralize pathogens in the environment.

### Summary of the Invention

The most important objective of the invention is to re-design the electric heater to heat indoor spaces and inactivate pathogens (virus, bacteria, and the like) by heating the ambient air to high temperatures while passing through the heater.

Another objective of the invention is controlling the air temperature and transit time through said heaters, to inactivate pathogens. The electric heater works in two different modes. In the first mode, it functions solely as a heater. In the second mode, it serves as a heater and an air purification system. A button on the system is used to switch the system to a heater or both a heater and an air purification system. If the device is selected to perform in both heater and air purification system mode, it runs above a predetermined temperature and below a predetermined airflow rate. In this way, pathogens are inactivated by being exposed to the specified temperature for the desired time.

Another objective of the invention is to direct the radiation generated by the electrically operated heaters into the heater, instead of directly reflecting it to the environment, so as to create high heat in the device. In this way, the air drawn into the heater by a fan reaches a high temperature while passing through the heater. The air passing through the heater below a specific speed allows pathogens to be exposed to high temperatures. As a result, the temperature of pathogens rises, their membrane integrity is disrupted due to the increase in membrane lipid fluidity. They are inactivated as a result of denaturing proteins and nucleic acids.

Although tungsten wire is generally used in electric heaters, carbon, chromium, iron, aluminum, and different metal alloys can also be used. Ceramic heaters with metal wires are used when the light emission is not visible. Ceramic heaters convert electrical energy into infrared radiation with 96% efficiency.

Another objective of the invention is to draw the heater by using a fan and making air passing through the heater and heating the passing air to at least 50 °C to inactivate the pathogens in the air.

Another objective of the invention is to inactivate the pathogens in the air entering from outside to inside in air conditioners used in buildings.

Another objective of the invention is to inactivate the airborne pathogens as they travel from the outside to inside in air conditioners used in buildings, buses, trams, planes, and similar vehicles used in personal and public transportation.

Another objective of the invention is that the air passing through the electric heater passes through a hollow cylinder without a pressure drop and the cylinder has a structure which ensures that air does not need to pass through very small pores. Therefore, resistance of air passing through the electric heater which is used as pathogen filter becomes much more lower so that the system may purify the air in a closed environment with a short time.

An objective of the invention is to protect against viruses, bacteria, and similar pathogens in indoor spaces with electrical heaters.

Another objective of the invention is that the air passing through the electric heater will reach a temperature of at least 50 °C to inactivate the viruses, bacteria, and similar pathogens.

Another objective of the invention is to increase the temperature and transit time of the air passing through the electric heater, such that the temperature of viruses and bacteria-like pathogens will disrupt their membrane integrity or DNA and/or RNA.

The structural and characteristic features and all advantages of the invention will be understood clearly by the figures given below and the detailed description written by referring to these figures. Therefore, the evaluation should be made by taking these figures and explicit explanations into consideration.

### Brief Description of the Drawings

**FIG. 1** is the drawing that illustrates the physical appearance of the electric heater airborne pathogen deactivation system of the invention.
**FIG. 2** is the drawing of front (2A), side section (2B), and rear (2C) views of the electric heater air purification system of the present invention.
**FIG. 3** is the drawing that illustrates the cross-sectional view containing the centrally heated air tube of the present invention's electric heater air purification system.
**FIG. 4** is the drawing showing the cross-sectional view of the electric heater air purification system of the invention.
**FIG. 5** is drawing the propellers and sectional views of the centrally heated air tube in the electric heater air purification system of the present invention.
**FIG. 6** shows the electric heating air purification system's cross-sectional view according to the surface heated air tube.

### Description of reference numbers

10) Electric heater pathogen air filter
11) Carrying handle
20) Control panel
21) On-off button
22) Temperature control button
23) Electric heater pathogen air filter button
24) Light indicator
25) Digital display
30) Centrally heated air tube
31) Hot air tube air outlet end
32) Hot air tube air inlet end
33) Air fan
34) Air grille
35) Support foot
36) Centrally heated air tube inner surface
37) Fiberglass
38) Centrally heated air tube outer surface
40) Quartz heater
41) Quartz tube
42) Resistive wire spiral
43) Ceramic carrier
44) Electrical connection terminal
50) Surface heated air tube
51) Quartz inner surface
52) Surface resistive wire spiral
53) Thermal insulation material
54) Steel tube

### Detailed Description of the Invention

The invention relates to the use of an electric heater as an airborne pathogen deactivation system at the same time. There is a carrying handle (11) on the electric heater pathogen air filter (10) to carry it and position it to the desired location easily. A control panel (20) is placed anywhere on the upper part of the electric heater pathogen air filter (10). The control panel (20) includes the digital display (25) showing the temperature, flow rate, and operating time of the air passing through the electric heater pathogen air filter (10). On-off button (21) enables the electric heater pathogen air filter (10) to start or stop its operation. Temperature control knob (22) adjusts the temperature inside the electric heater air purification system (10), while the electric heater pathogen air filter (10) is operating in the electric heater mode, the air purification system mode is active or passive at that time. The electric heater pathogen air filter button (23) enables the electric heater to be turned on as an air purification system. The light indicator (24) indicating whether the pathogen air cleaning filter is active or passive, which turns on if it is active and turns off if it is inactive. The electric heater pathogen air filter (10) can be used only as a heater if the electric heater pathogen air filter button (23) is off. The electric heater pathogen air filter (10) can be used as an electric heater and air purification system when the electric heater pathogen air filter button (23) is turned on. In this case, the light indicator (24) on the control panel (20) turns on and shows that the electric heater pathogen air filter (10) is operating in pathogen air purification mode. The electric heater pathogen air filter (10) in the pathogen air purification mode enables heating a closed environment with electricity. At the same time, it ensures the inactivation of viruses and similar pathogens in the air.

The electric heater pathogen air filter (10) of the present invention has at least one air tube positioned inside its outer body, at least one heat source emitting infrared, and at least one propeller that allows the outside air be taken in the air tube. Said air tube can be a centrally heated air tube (30) or a surface heated air tube (50). When a centrally heated air tube (30) is used, a metallic heat source such as resistance wire or an insulating material such as porcelain, ceramic, or quartz, a quartz heater (40) that emits infrared radiation is centrally located in the centrally heated air tube (30). In the surface heated air tube (50), at least one surface resistive wire spiral (52) is wrapped with resistance wire coils. The surface heated air tube (50) is made of an insulating material such as porcelain or ceramic, or quartz. The quartz heater (40) mentioned here, and the quartz tube (41) to be mentioned later are used as terminology. The material of the quartz heater (40) consists of a quartz tube (41) and a resistive wire spiral (42) inside the quartz tube (41). The heater can also be made of ceramic or porcelain material.

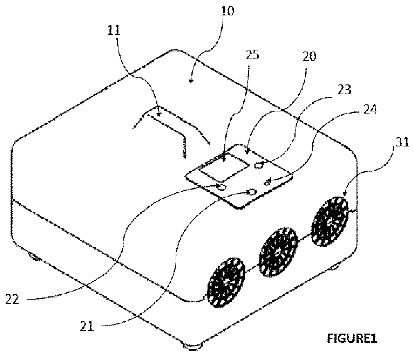

In Figure 1, the physical appearance of the electric heating air purification system (10) is given. The hot air tube air outlet end (31) is seen on the side of the said electric heater pathogen air filter (10).

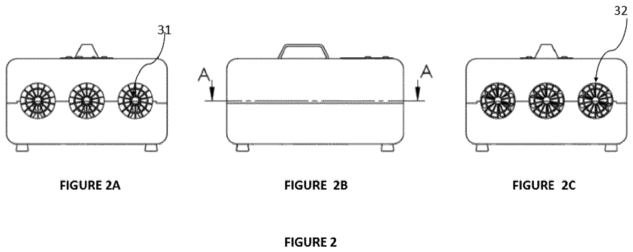

Figure 2 shows the front and rear views of the electric heater pathogen air filter (10). The hot air tube air outlet end (31) is positioned on one side edge of the electric heater pathogen air filter (10), and hot air tube air inlet end (32) is placed on the other side edge. The ambient air enters into the electric heater pathogen air filter (10) through the hot air tube air inlet end (32), and by increasing the temperature of the air in the system inactivates, bacteria, viruses, molds, fungi, etc. Then the air leaves the electric heater pathogen air filter (10) through the hot air tube air outlet end (31).

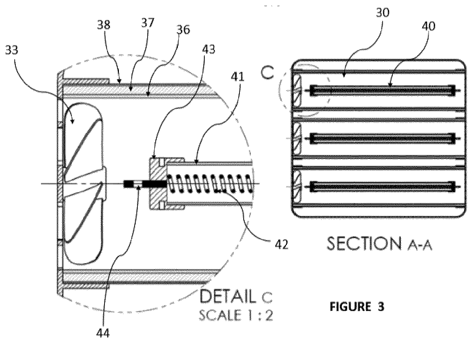

A-A side section of the electric heater pathogen air filter (10) is given in Figure 2. The form of the centrally heated air tube (30) is shown in Figure 3. In the section, the centrally heated air tube (30) is heated by a centrally placed quartz heater (40), and the air fan (33) are seen. Inside the electric heater pathogen air filter (10), there may be more than one central heated air tube (30) and more than one air fan (33). Said centrally heated air tubes (30) can be positioned side by side or on top of each other in the electric heater pathogen air filter (10). At the center of each centrally heated air tube (30), there is at least one heater element, such as a quartz heater (40) and at least one air fan (33). At the center of the centrally heated air tube (30) there is enough space to accommodate at least one quartz heater (40), and the inner surface of the long sides consists of three layers. The air fan (33) is positioned on the side edge of the centrally heated air tube (30) to let air into the tube. Said layers, start from the center of the centrally heated air tube (30) towards the outer environment, respectively: the centrally heated air tube inner surface (36) of the centrally heated air tube (30), the fiberglass (37) providing insulation on the centrally heated air tube inner surface (36) of the centrally heated air tube, and the centrally heated air tube outer surface (38) on the fiberglass (37). As shown in Figure 3, the quartz heater (40) inside the centrally heated air tube (30) consists of a quartz tube (41) and the resistive wire spiral (42) inside it. The quartz heater (40) is connected to the ceramic carrier (43), and its electrical connection is made at the electrical connection terminal (44). The air fan (33) on the side of the centrally heated air tube (30) provides the outside air to enter the centrally heated air tube (30).

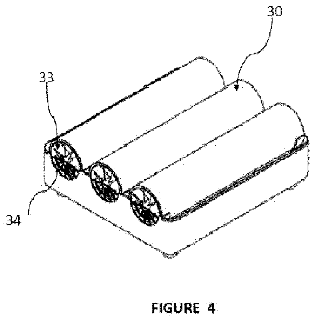

Figure 4 shows the air fan (33) on the side edges of the centrally heated air tube (30) and the air grille (34) positioned at the inlet of the centrally heated air tube (30) to prevent foreign objects from hitting the propeller.

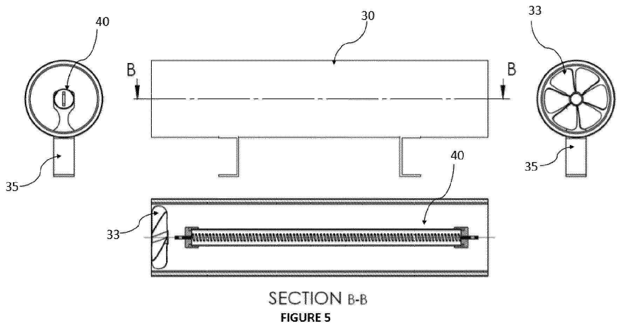

In Figure 5, if the centrally heated air tubes (30) are lined up side by side inside the electric heater pathogen air filter (10), each centrally heated air tube (30) will be placed in the outer body of the electric heater pathogen air filter (10) using at least one support foot (35) of the centrally heated air tube (30). In Figure 5, the quartz heater (40) located inside the centrally heated air tube (30), the air fan (33) on either side of the centrally heated air tube (30) are shown as horizontally positioned.

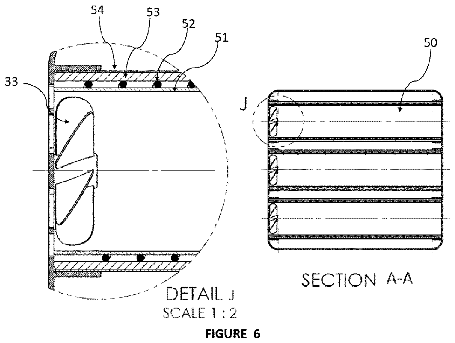

In another embodiment of the present invention, the surface heated air tube (50) is used instead of the centrally heated air tube (30). A-A side section view of the surface heated air tube (50) of the electric heater pathogen air filter (10) is illustrated in Figure 6 obtained from the A-A section of Figure 2. The surface heated air tube (50) and the air fan (33) can be seen from the section's surface. Inside the electric heater pathogen air filter (10), there are more than one surface heated air tubes (50) and more than one air fan (33). Said surface heated air tubes (50) can be positioned side by side or on top of each other in the electrical heating air purification system (10). There is at least one air fan (33) within the surface heated air tube (50). The surface heated air tube (50) has heaters wrapped on the long sides of the surface heated air tube (50). The air fan (33) is also positioned on the side edge of the surface heated air tube (50) to let in the air inside the surface heated air tube (50). The surface heated air tube (50) is heated from the quartz inner surface (51) by the wrapped resistance heater. Surface heated air tube (50) having a quartz inner surface (51) has at least one surface resistive wire spiral (52) wrapped on its long edges. There is thermal insulation material (53) on the quartz inner surface (51) to keep the heat inside the inner quartz surface (51) of the surface heated air tube (50). The surface heated air tube (50), the outermost part is the steel tube (54) and provides mechanical strength against the outside.

The electric heater air purification system (10) inactivates airborne bacteria, viruses, mold, fungus, etc., taken inside by the air fan (33) into either the centrally quartz heater (40) or surface heated air tube (50). One way of inactivating airborne pathogens is the increasing temperature of the dry air. Airborne pathogens have different resistance to heat. Bacillus Subtilis is a gram-positive bacterium. It has a higher resistance to heat than Escherichia Coli, which is a gram-negative bacterium. Gram-positive bacteria have a higher resistance to temperature than gram-negative bacteria.
Similarly, viruses are resistant to heat according to their structure; for example, viruses with lipid envelopes are less resistant to heat than viruses without a lipid envelope. The most crucial parameters in the deactivation of viruses are inactivation energy, exposure time, and hot air temperature. In this respect, the inactivation temperature and duration vary with the inactivation energy of viruses.

The inactivation of a airborne pathogen by the electric heater pathogen air filter (10), depends on the inactivation energy of the pathogen, the air temperature in the centrally heated air tube (30) or the surface heated air tube (50), and the residence time of the pathogens inside the tube. Therefore, a pathogen has a minimum residence time inside a tube (centrally heated air tube (30) or surface heated air tube (50)) depending on the air temperature inside the tube and inactivation energy of airborne pathogen while passing through the electric heater pathogen air filter (10) to be deactivated. This period should be at least 0.01 second.

Airborne pathogen-free air leaves the electric heater pathogen air filter (10) from the hot air tube air outlet end (31) with a temperature above 50 °C with the pathogens' residence time in the tubes above 0.01 s. The electric heater pathogen air filter (10) ensures airborne pathogens inactivation by keeping the temperature of the centrally heated air tube (30) or the surface heated air tube (50) above a specific temperature. The residence time of the air inside the tube (the centrally heated air tube (30) or the surface heated air tube (50)) is provided to inactivate the pathogens by varying the air fan (33) flow. The pathogen-free air leaves the electric heater pathogen air filter (10) at the hot air tube air outlet end (31) at a temperature of at least 50 °C. Therefore, pressing the electric heater pathogen air filter button (23) to inactivate the electric heater pathogen air filter (10) provides the tube's temperature and air residence time conditions.

The centrally heated air tube (30) or the surface heated air tube (50) of the electric heater pathogen air filter (10) can be designed in different ways to increase the air temperature more effectively. It comprises at least coiled heating resistance vire positioned perpendicular to the airflow direction inside the centrally heated air tube (30) or the surface heated air tube (50). Thus, the air coming from the external environment entering into the centrally heated air tube (30) or the surface heated air tube (50) passes through the centrally heated air tube (30) or the surface heated air tube (50) and hits the heating elements such as coiled resistance vire.

In the electric heater pathogen air filter (10) of the present invention, as described above, centrally heating or surface heating systems can be performed for the air to reach high temperatures and effectively deactivating the pathogens. The surface heated air tube (50) can be arranged in different geometries. They can be straight cylindrical or have L or U or S forms. Besides, high temperatures inside the tube (centrally heated air tube (30) or surface heated air tube (50)), where the air can be heated by exposing the centrally heated air tube (30) or the surface heated air tube (50) and increase the contact with the hot surface. Air fins may also be used inside the tubes to increase air temperature more effectively by increasing the tubes' hot surface.

One way to heat the air more efficiently is to put the heaters perpendicular to the airflow. These heaters can be electric resistance wires or metal tubes with an electric resistance wire inside. In the electric heater pathogen air filter (10) of the present invention, resistances are positioned perpendicular to the airflow in a tube so that the air reaches high temperatures.

In order for the electric heater pathogen air filter (10) of the present invention to deactivate pathogens in the air, the temperature of the heater inside the centrally heated air tube (30) or surface heated air tube (50) should increase to at least 100 °C and transit time of the pathogen in the hot air tube (centrally heated air tube (30) or surface heated air tube (50)) should be above 0.1 seconds. These conditions are minimum, and the infrared radiation source's temperature should be minimum 100 °C and a maximum of 2000 °C according to the pathogen's deactivation energy to be deactivated. In this temperature range, the time the air spends in the tube (centrally heated air tube (30) or surface heated air tube (50) should be between 0.1 seconds and 30 minutes to deactivate pathogens.

The electric heater pathogen air filter (10) deactivate viruses, bacteria, and similar airborne pathogens by taking the air inside the air tubes and increasing the temperature to at least 50 °C. While the said electric heater pathogen air filter (10) serves only as an electric heater, the pathogen filter is activated, ensuring that the electric heater also has the pathogen air purification feature. Electric heater pathogen air filter (10) has at least one central heated air tube (30) or one surface heated air tube (50) to heat airborne pathogens.

Central heated air tube (30) or surface heated air tube (50) has at least one air fan (33) that enables the entrance of the outdoor air into the tubes. Electric heater pathogen air filter (10) has at least one temperature control button (22) that allows temperature adjustment. Electric heater pathogen air filter (10) has an electric heater pathogen air filter button (23) of the electric heater, which enables the pathogen air purification filter mode to be activated or deactivated while the electric heater mode of the electric heater pathogen air filter (10) is running. The system contains at least one infrared emission source that provides enough temperature to degrade or denature proteins and nucleic acids.

### REFERENCES

Bertrand, I., Schijven, J. F., Sanchez, G., Wyn-Jones, P., Ottoson, J., Morin, T., ... Gantzer, C. (2012). The impact of temperature on the inactivation of enteric viruses in food and water: A review. Journal of Applied Microbiology, 112(6), 1059-1074. https://doi.org/10.1111/j.1365-2672.2012.05267.x
Damit, B., Wu, C. Y., & Yao, M. (2013). Ultra-high temperature infrared disinfection of bioaerosols and relevant mechanisms. Journal of Aerosol Science, 65, 88-100. https://doi.org/10.1016/j.jaerosci.2013.07.010
Darnell, M. E. R., Subbarao, K., Feinstone, S. M., & Taylor, D. R. (2004). Inactivation of the coronavirus that induces severe acute respiratory syndrome, SARS-CoV. Journal of Virological Methods, 121(1), 85-91. https://doi.org/10.1016/j.jviromet.2004.06.006
Grinshpun, S. A., Adhikari, A., Li, C., Yermakov, M., Reponen, L., Johansson, E., & Trunov, M. (2010). Inactivation of aerosolized viruses in continuous air flow with axial heating. Aerosol Science and Technology, 44(11), 1042-1048. https://doi.org/10.1080/02786826.2010.509119
Hessling, M., Hoenes, K., & Lingenfelder, C. (2020). Selection of parameters for thermal coronavirus inactivation - a data-based recommendation. GMS Hygiene and Infection Control, 15, Doc16. https://doi.org/10.3205/dgkh000351
Jung, J. H., Lee, J. E., & Kim, S. S. (2009). Thermal effects on bacterial bioaerosols in continuous air flow. Science of the Total Environment, 407(16), 4723-4730. https://doi.org/10.1016/j.scitotenv.2009.05.008
Jung, J. H., Lee, J. E., Lee, C. H., Kim, S. S., & Lee, B. U. (2009). Treatment of fungal bioaerosols by a high-temperature, short-time process in a continuous-flow system. Applied and Environmental Microbiology, 75(9), 2742-2749. https://doi.org/10.1128/AEM.01790-08
Pablo Jeremías, P. G., Hernandez, M., Tello, M., Ruiz Sanjuán, C., Campo-Arias, A., Herazo, E., ... Hermiyanty, Wandira Ayu Bertin, D. S. (2013). 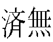 No Title No Title. *Intangible Capital, 11*(1), 10. https://doi.org/10.1007/978-3-319-64834-7
Pastorino, B., Touret, F., Gilles, M., & Lamballerie, X. De. (2020). Samples: What Protocols for Biosafety,. 6-13.
Yap, T. F., Liu, Z., Shveda, R. A., & Preston, D. J. (2020). A predictive model of the temperature-dependent inactivation of coronaviruses. Applied Physics Letters, 117(6), 060601. https://doi.org/10.1063/5.0020782

## Claims

1. An electric heater pathogen air filter (10) which enables deactivation of viruses, bacteria and similar pathogens in the air at high temperature, that has a characteristics of an electric heater and also a pathogen purification filter; **characterized by** comprising
• At least one centrally heated air tube (30) or at least one surface heated air tube (50) that enables the air to enter the electric heater pathogen air filter (10); at least one air fan (33) that enables intake of the outside air into the centrally heated air tube (30) or the surface heated air tube (50); a temperature control button (22) that enables temperature adjustment by controlling the temperature inside centrally heated air tube (30) or surface heated air tube (50); an electric heater pathogen air filter button (23) which enables the pathogen air purification filter mode to be activated or deactivated at the same time when the electric heater mode is running,
• at least one infrared emission source located in the centrally heated air tube (30) or the surface heated air tube (50) which disrupts the membrane integrity of virus and bacteria-like pathogens in the air or provides enough temperature to denature protein and nucleic acids of the pathogens.

2. An electrical heater pathogen air filter (10) according to Claim 1, **characterized in that** the infrared emission source located in the centrally heated air tube (30) or the surface heated air tube (50) is a quartz heater (40) or a surface resistive wire spiral (52) on the surface heated air tube (50).

3. An electrical heater pathogen air filter (10) according to Claim 1 or 2, **characterized in that** the quartz heater (40) consisting of the quartz tube (41) and resistive wire spiral (42) is positioned at the center of inside of the centrally heated air tube (30).

4. An electrical heater pathogen air filter (10) according to Claim 1 or 3, wherein the quartz tube (40) inside the centrally heated air tube (30) is made of an insulating material which is porcelain or ceramic.

5. An electrical heater pathogen air filter (10) according to Claim 1, **characterized in that** the centrally heated air tube (30) has an centrally heated air tube inner surface (36) that the long sides of it are made of metal, a fiberglass (37) providing heat insulation on the inner surface of the centrally heated air tube inner surface (36) and centrally heated air tube outer surface (38) on the fiberglass (37).

6. An electrical heater pathogen air filter (10) according to Claim 1 or 2, **characterized in that** the surface heated air tube (50) has more than one surface resistive wire spiral (52) wrapped on long side edges.

7. An electrical heater pathogen air filter (10) according to Claim 1, **characterized in that** the surface heated air tube (50) which is heated from the surface, has a thermal insulation material (53) on the top of the surface resistive wire spiral (52) wrapped on the quartz inner surface (51) and a steel tube (54) on the outer part that provides mechanical strength against the outside.

8. An electrical heater pathogen air filter (10) according to Claim 1, wherein the temperature of the hot air tube air outlet end (31) of the centrally heated air tube (30) is at least 50 °C.

9. An electrical heater pathogen air filter (10) according to Claim 1 or 6, wherein the surface heated air tube (50) heated from the surface on which the surface resistive wire spiral (52) is wrapped, is made of an insulating material which is quartz or porcelain or ceramic.

10. An electrical heater pathogen air filter (10) according to Claim 1, **characterized in that** temperature of the infrared radiation source which is placed inside the centrally heated air tube (30) or surface heated air tube (50) is at least 100 °C and maximum 2000 °C, in order to be used as a pathogen air filter.

11. An electrical heater pathogen air filter (10) according to Claim 1, **characterized in that** the air transition time in the centrally heated air tube (30) or surface heated air tube (50) in the electric heater pathogen air filter (10) is at least 0.1 seconds and 30 minutes at the most, in order to be used as an pathogen air filter.

12. An electrical heater pathogen air filter (10) according to Claim 1, wherein the hot air tube has a flat cylindrical or "L" or "U" or "S" form or a geometric form to increase the contact between the air and hot surfaces in the tube.

13. An electrical heater pathogen air filter (10) according to Claim 1, wherein the centrally heated air tube (30) or surface heated air tube (50) comprises hot grills positioned perpendicular to the flow direction of the air flow which increases the contact of the air entering the centrally heated air tube (30) or the surface heated air tube (50) with the hot surfaces of the centrally heated air tube (30) or the surface heated air tube (50).

## Amended claims

### Amended claims in accordance with Rule 137(2) EPC.

1. An electric heater which enables deactivation of viruses, bacteria and similar pathogens in the air at high temperature; **characterized by** comprising
• A control panel (20) includes the digital display (25) showing the room temperature, flow rate, and operating time of the air passing through the electric heater (10) and on-off button (21) enables the electric heater to start or stop its operation,
• A temperature control button (22) positioning on the control panel (20) and adjust the power of the electric heater (10) wherein operating in the electric heater mode,
• An electric heater button (23) positioning on the control panel (20) and which enables the pathogen air purification filter mode to be activated or deactivated wherein turned on as an air purification system,
• At least one centrally heated air tube (30) or one surface heated air tube (50) that enables the air to enter the electric heater (10),
• At least one air fan (33) that enables intake of the outside air into the centrally heated air tube (30) or the surface heated air tube (50),
• At least one infrared emission source located in the centrally heated air tube (30) or the surface heated air tube (50),
• A hot air tube air outlet end (31) positioned on the side of the said electric heater (10).

2. An electrical heater according to Claim 1, **characterized in that** the centrally heated air tube (39) has one quartz heater (40) located centrally or the surface heated air tube (50) heated by a surface resistive wire spiral (52) on the surface heated air tube (50)

3. An electrical heater according to Claim 1 or 2, **characterized in that** the quartz heater (40) consisting of the quartz tube (41) and resistive wire spiral (42) the inside of the centrally heated air tube (30).

4. An electrical heater according to Claim 1 or 3, wherein the quartz heater (40) inside the centrally heated air tube (30) is made of an insulating material which is porcelain material or ceramic.

5. An electrical heater according to Claim 1, **characterized in that** the centrally heated air tube (30) has a centrally heated air tube inner surface (36) that the long sides of it are made of metal.

6. An electrical heater according to Claim 2, **characterized in that** the surface heated air tube (50) has more than one surface resistive wire spiral (52) wrapped on long side edges.

7. An electrical heater according to Claim 2, **characterized in that** the surface heated air tube (50) which is heated from the surface, has a thermal insulation material (53) on the top of the surface resistive wire spiral (52) wrapped on the quartz inner surface (51) and a steel tube (54) on the outer part that provides mechanical strength against the outside.

8. An electrical heater according to Claim 1, wherein the temperature of the hot air tube air outlet end (31) of the centrally heated air tube(30) is at least 50 °C measured by the temperature control button (22).

9. An electrical heater according to Claim 1 or 6, wherein the surface heated air tube (50) heated from the surface on which the surface resistive wire spiral (52) is wrapped, is made of an insulating material which is quartz or porcelain or ceram ic.

10. An electrical heater according to Claim 1, **characterized in that** temperature inside the centrally heated air tube (30) or surface heated air tube (50) is at least 100 °C and maximum 2000 °C measured by the temperature control button (22), in order to be used as a pathogen air filter.

11. An electrical heater according to Claim 1, **characterized in that** the air transition time in the centrally heated air tube (30) or surface heated air tube (50) in the electric heater (10) is at least 0.1 seconds and 30 minutes at the most, in order to be used as a pathogen air filter.

12. An electrical heater according to Claim 1, wherein the hot air tube has a flat cylindrical or "L" or "U" or "S" form or a geometric form to increase the contact between the air and hot surfaces in the tube.

13. An electrical heater according to Claim 1, wherein the centrally heated air tube (30) or surface heated air tube (50) comprises hot grills positioned perpendicular to the flow direction of the air flow which increases the contact of the air entering the centrally heated air tube (30) or the surface heated air tube (50) with the hot surfaces of the centrally heated air tube (30) or the surface heated air tube (50)
